# EUROPEAN PATENT APPLICATION

(11) **EP 1 403 237 A1**
(43) Date of publication of application: **31.03.2004**
(21) Application number: 02745835.5
(22) Date of filing: 04.07.2002
(51) Int. Cl.: C07C 7/14, C07C 15/24, C07C 15/28, C07C 17/392, C07C 25/18, C07C 25/22, C07D 215/28, C07F 5/06

(54) **DEVICE AND METHOD FOR REFINING SOLID MATERIAL**

(30) Priority: 05.07.2001 JP 2001204604
(71) Applicant: ASAHI GLASS COMPANY LTD., Tokyo 100-8405 (JP)
(72) Inventor: CHIBA, Kazunori, Asahi Glass Company, Limited, Kanagawa 221-8755 (JP); MUROFUSHI, Hidenobu, Asahi Glass Company, Limited, Kanagawa 221-8755 (JP)
(74) Representative: Müller-Boré & Partner Patentanwälte
(86) International application number: PCT/JP2002/006801
(87) International publication number: WO 2003/004139

(57) **Abstract**

To provide a device for refining a solid material and a process for refining it, whereby from the supply of the material to the operation for recovery of crystals can be carried out batch-wisely or continuously, and inclusion of foreign matters such as particles can be efficiently prevented. A device for refining an evaporable or sublimable solid material, which comprises a housing, and at least one rotatable roller for evaporation and at least one rotatable roller for precipitation, installed in the housing.

## Description

### TECHNICAL FIELD

The present invention relates to a device and process for refining a solid material. More particularly, it relates to a device and process for refining a solid material, wherein an evaporation section and a solid precipitation section are constituted by rotatable rollers and whereby a batch-wise or continuous operation is possible, and inclusion of foreign fine particles can be efficiently prevented.

### BACKGROUND ART

As a common method for refining a solid material, recrystallization may be mentioned. Further, a method for refining such as distillation or sublimation may also be employed in a case where the solid material is liquefiable and evaporable, or the solid material is sublimable. In the refining by recrystallization, high purification is possible. However, by recrystallization, there may be a case where continuous recovery of crystals tends to be difficult, or removal of a solvent deposited on recovered crystals tends to be difficult. Whereas, by sublimation, there will be a problem such that the sublimation speed is slow, or continuous recovery of crystals tends to be difficult. Further, for distillation of a solid material, a Kouguel distilling machine may, for example, be used in a laboratory scale. However, such distillation has problems such that recovery of crystals tends to be difficult, it is necessary to open the apparatus at the time of the recovery and to scrape out the crystals, whereby inclusion of foreign matters such as particles is unavoidable, and the refining efficiency is not necessarily high. Among these refining methods, in a case where a material which is likely to undergo decomposition at a high temperature or in the vicinity of the boiling point, is to be refined by distillation or sublimation, an operation under reduced pressure is effective for high purification, rather than under atmospheric pressure. However, under reduced pressure, continuous or semi-continuous recovery of crystals tends to be difficult. Accordingly, for refining an evaporable or sublimable solid material, it is desired to develop a device whereby the solid material can be continuously refined to a high purity.

As a conventional industrial device for refining by sublimation, a device disclosed in JP-A-2-16166 is known. However, in this device, heating of the solid to be sublimated is by radiation heat transmission, whereby the transmission efficiency is low, and the required calorie per unit mass of the solid material is large, and thus, it is of a large energy consumption type. Further, the drum to recover crystals will be in contact with the blade member to scrape off the crystals, whereby dusting and inclusion of foreign matters in the recovered sublimation refined pigment are likely to occur. Further, the recovery method of the refined pigment is non-continuous, whereby continuous refining is difficult. Furthermore, manual operation is essential, and it is necessary to open the system at the time of the recovery, whereby inclusion of foreign matters such as particles is unavoidable, whereby it is difficult to obtain a high level of refining.

On the other hand, with the sublimation refining device disclosed in JP-A-2000-93701, a continuous operation is difficult in the supply of the material and in the recovery of the refined product. Further, this device has a problem that the heat transmission area per unit volume of the material to be sublimated, is small, and the treating capacity is small. Further, the recovery method for the refined product is non-continuous, and a manual operation is essential, and it is necessary to open the system at the time of the recovery, whereby inclusion of foreign matters such as particles is unavoidable, and it is difficult to obtain highly refined crystals of a high purity.

It is an object of the present invention to provide a highly efficient device and process for refining a solid material, whereby a material which is a solid compound and which can be refined by evaporation or sublimation, is permitted to form crystals of high purity in a thermally stable state, and from the supply of the material to the recovery operation of crystals can be carried out in a series of operations batch-wisely or continuously, and whereby inclusion of e.g. particles can be prevented.

### DISCLOSURE OF THE INVENTION

The present invention provides a device for refining an evaporable or sublimable solid material, which comprises a housing, and at least one rotatable roller for evaporation and at least one rotatable roller for precipitation, installed in the housing. By this refining device, the solid material can be refined highly efficiently and in high purity.

Here, it is preferred that the distance between the roller for evaporation and the roller for precipitation is adjustable. By this embodiment, the positions of the two rollers suitable for the solid material to be refined can be selected, whereby the refining efficiency can be improved.

Further, it is preferred that the surface of the roller for evaporation and/or the roller for precipitation has an irregularity. By this embodiment, the evaporation efficiency, the sublimation efficiency and the precipitation efficiency can be improved, and the overall refining efficiency can be improved. Especially when the surface of the roller for evaporation has an irregularity, the efficiency in stirring of the solid material can be improved, and evaporation or sublimation can be stabilized.

Further, it is preferred that the roller for evaporation and the roller for precipitation can be heated and/or cooled. By this embodiment, the evaporation or sublimation can be carried out stably and in a large quantity, and further, the efficiency in precipitation can be increased, and the overall refining efficiency can be improved.

Further, it is preferred that the refining device is further provided with a scraping means in the vicinity of the roller for precipitation, wherein there is a space left between the surface of the roller for precipitation and the forward end of the scraping means. By this embodiment, it is possible to prevent inclusion of particles which is otherwise likely to take place by abrasion between the roller for precipitation and the scraping means.

Further, it is preferred that the housing is heated to prevent deposition of crystals onto the inner wall of the housing. By this embodiment, precipitation of the solid material on other than the roller for precipitation can be prevented, whereby the overall refining efficiency can be improved.

Further, the present invention provides a process for refining a solid material, which comprises batch-wisely or continuously evaporating or sublimating a solid material deposited on the surface of a rotatably installed roller for evaporation; batch-wisely or continuously precipitating the evaporated or sublimated material on a rotatably installed roller for precipitation; batch-wisely or continuously scraping off crystals precipitated on the surface of the roller for precipitation, at a scraping section; and batch-wisely or continuously discharging the crystals. By this process, the solid material can be refined highly efficiently and in high purity.

### BRIEF DESCRIPTION OF THE DRAWING

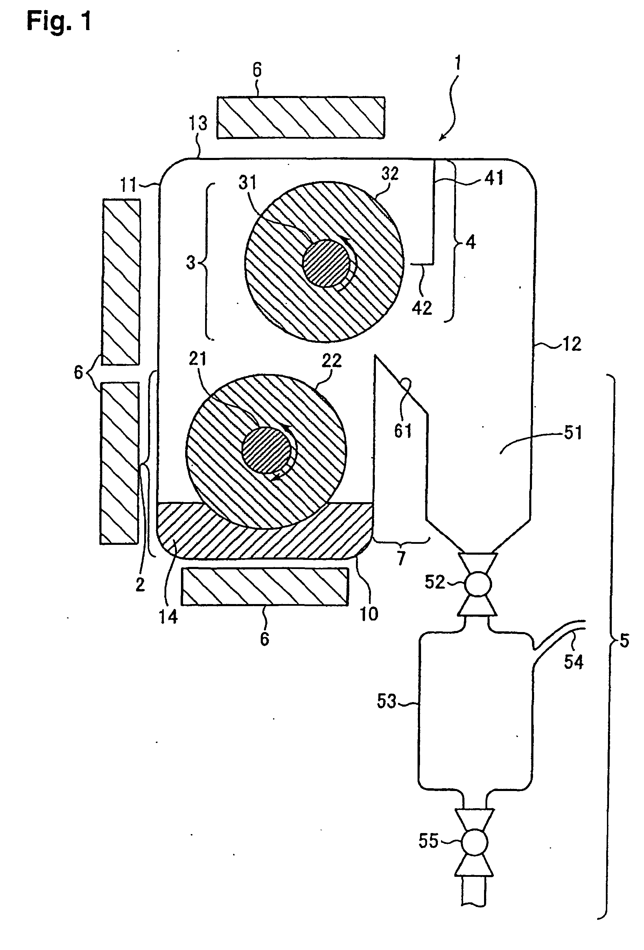

Fig. 1 is a vertical cross-sectional view of the essential portion of the refining device of the present invention (a schematic view of an embodiment).

### Description of reference numerals

1: housing, 2: evaporation section, 3: solid precipitation section, 4: scraping section, 5: solid discharge section, 6: heater, 7: depressed portion, 10: bottom wall, 11, 12: side wall, 13: top wall, 14: material, 21: rotational axis of the roller for evaporation, 22: roller for evaporation, 31: rotational axis of the roller for precipitation, 32: roller for precipitation, 41: scraper, 42: forward end, 51: storage section, 52: upper discharge valve, 53: discharge section, 54: pressure adjusting line, 55: lower discharge valve, 61: guide wall for crystals.

### BEST MODE FOR CARRYING OUT THE INVENTION

In the present invention, "evaporation or sublimation" means a case where the solid material once melted, is evaporated ("evaporation" meaning a change of state from a liquid phase to a gas phase (inclusive of boiling)) and a case where the solid material is sublimated without being melted ("sublimation" meaning a change of state from a solid phase to a gas phase), but these are not clearly distinguished, and the expression includes a case where they occur simultaneously. Further, in this specification, "evaporation or sublimation from the surface of the roller for evaporation" means evaporation or sublimation from the surface of the roller for evaporation of the solid material, but it includes a case where the solid material is evaporated or sublimated from the surface and the interior of the solid material.

In the present invention, "roller for evaporation" means a roller to evaporate or sublimate the solid material, and "evaporation section" means part of the device to evaporate or sublimate the solid material, equipped with the roller for evaporation.

In the present invention, "roller for precipitation" means a roller to precipitate the solid material, and "solid precipitation section" means part of the device to precipitate the solid material, equipped with the roller for precipitation. It is preferred that in the solid precipitation section, a scraping section is provided in the vicinity of the roller for precipitation.

In the present invention, "housing" means the exterior frame structure of the device comprising the outer walls (the bottom wall, the top wall and the side walls) of the refining device.

The refining device of the present invention comprises the housing, the evaporation section and the solid precipitation section, and in each of the evaporation section and the solid precipitation section, at least one rotatable roller is installed. Namely, each of the evaporation section and the solid precipitation section in the present invention is constituted by at least one rotatable roller. Accordingly, it is not limited to a structure wherein one roller is installed in each of the evaporation section and the solid precipitation section, and a plurality of rollers may be installed in the evaporation section and the solid precipitation section, respectively. Further, the number of rollers may not necessarily be the same in the evaporation section and in the solid precipitation section, and the respective sections may be constituted by different numbers of rollers. By such a construction, the roller for evaporation is capable of evaporating or sublimating the solid material constantly and in a constant amount. Further, on the roller for precipitation, the solid material will be continuously precipitated as crystals. Further, at the scraping section which will be described hereinafter, such precipitated crystals can be continuously scraped off.

The shapes of the roller for evaporation at the evaporation section and the roller for precipitation at the solid precipitation section, are not particularly limited. For example, a cylindrical form or a columnar form may be mentioned. The surface structures of the roller for evaporation and the roller for precipitation are not particularly limited. On the surface of these rollers, an irregularity such as a groove may be formed. As examples of the shape of the irregularity, a linear shape, a helical shape, a wave shape and a net shape may, for example, be mentioned, and a shape to increase the surface area required for evaporation, sublimation or precipitation, is preferred. By forming the irregularity on the surface of the roller for evaporation, the solid material to be evaporated or sublimated tends to readily deposit on the roller for evaporation (including a case where the solid material is mounted in a recessed portion of the surface shape of the roller for evaporation), and the heat transmission area will increase to accelerate evaporation or sublimation. Further, by forming the irregularity on the surface of the roller for precipitation, the area will be increased, whereby precipitation (deposition) of crystals will be accelerated. Further, in the case of a sublimable material, the solid material can efficiently be stirred, whereby sublimation will proceed constantly. Further, the cross-sectional shape of such a roller (when considered without taking the irregularity into consideration) can be freely selected from a circular shape, a polygonal shape, etc., depending upon the material to be evaporated or sublimated, but particularly preferred is a circular shape.

The roller for evaporation and the roller for precipitation are preferably of a structure such that the temperature is adjustable by heating or cooling. Here, the roller for evaporation is particularly preferably of a structure which can be heated. As such a structure, a double tubular structure, a hollow structure may be employed, as the case requires. The heating source may, for example, be an electric heater, steam or electromagnetic induction heating. As the cooling source, a cooling medium such as flon, ammonia or brine, or a water, may, for example, be mentioned. Usually, by adjusting the pressure and the temperature of the entire device, the evaporation amount or the sublimation amount is adjusted. In the present invention, by directly heating the roller for evaporation, the heating efficiency will be improved, whereby the material can be evaporated or sublimated constantly and in a large amount. Further, by heating or cooling the roller for precipitation, the precipitation efficiency can be improved. Here, the temperature of the roller for precipitation is preferably controlled precisely in order to adjust the precipitation speed of crystals. Namely, the roller for precipitation is set to be slightly lower than the temperature in the housing or the temperature of the roller for evaporation. At that time, if the roller for precipitation is excessively cooled, fine crystals will be precipitated, and the crystal precipitation speed may tend to decrease as a whole. When the roller for precipitation is maintained at a predetermined temperature, large crystals are likely to grow, whereby the crystal growth rate can be improved as a whole. Accordingly, the temperature for the roller for precipitation is preferably controlled to be lower by from 5 to 50°C, particularly preferably lower by from 20 to 30°C, than the temperature of the roller for evaporation.

Further, the driving method to rotate the roller for evaporation and the roller for precipitation and the sealing method for the shafts, etc., may suitably be selected depending upon the particular purpose. The rotational direction and the rotational speed of the roller for evaporation and the roller for precipitation are preferably controllable independently of each other. Further, when a higher purification is intended, it is impossible to prevent inclusion of foreign matters such as particles by usual mechanical sealing or the like, since dusting is thereby substantial, and a driving method and a sealing method, such as magnet driving, etc., are preferred.

The material for the roller for evaporation and the roller for precipitation can be selected depending upon the conditions for evaporation, sublimation or precipitation, the corrosion resistance, etc. among metal materials, ceramic materials, glass materials, fluorine resins, etc.

With respect to positioning of the roller for evaporation and the roller for precipitation in the present invention, it is preferred that the roller for evaporation is disposed at a lower portion of the housing which will be described hereinafter, and the roller for precipitation is disposed in the upper portion thereof. It is particularly preferred that the roller for precipitation is disposed diagonally above the roller for evaporation. By such disposition, continuous evaporation, sublimation or precipitation of the solid material will be possible, and highly efficient refining will be possible. Further, disposition of the roller for evaporation and the roller for precipitation (hereinafter referred to as "roller distance") is preferably such that the mutual positions are adjustable. As a method for adjusting the roller distance, it is preferred that the roller for evaporation and the roller for precipitation are respectively independently movable against the housing, so that the optimum positions can be selected. Further, in a case where the positions of the roller for evaporation and the roller for precipitation are fixed against the housing, the roller distance may be adjusted by providing dividable portions in the housing and inserting a spacer. As an example of the dividable portions in the housing, a structure may be mentioned in which two engaging flanges are provided in the housing, and between such flanges, a sealing portion such as an o-ring is provided. In such a case, by inserting a spacer of a suitable shape between the flanges, the roller distance can be increased.

By controlling the surface areas, the surface shapes, the rotational speeds, the roller distance and the temperatures of the roller for evaporation and the roller for precipitation, it is possible to control the recovery amount (the recovery rate) of crystals. Further, by adjusting the recovery rate, the purity can also be controlled. The refining process of the present invention has a high degree of freeness in this respect, and can be adjustable to any level from refining of a small amount in a laboratory to a large amount of an industrial scale. Further, the refining device of the present invention is suitable for refining operations for materials different in e.g. the evaporation conditions, sublimation conditions, vapor densities, etc.

The refining device of the present invention is preferably provided with a scraping means (hereinafter sometimes referred to also as "a scraping member") in the vicinity of the roller for precipitation. Such a scraping means is not particularly limited, and a scraper, a brush or the like may be mentioned. Among them, a scraper is particularly preferred as the scraping means. In a case where a scraping member is provided, it is preferred that there is a space between its forward end and the surface of the roller for precipitation. By providing such a space, it is possible to prevent inclusion of particles which may otherwise be formed by the contact of the scraping member with the roller for precipitation. The space (distance) between the roller for precipitation and the forward end of the scraping member is preferably from 0.01 to 30 mm, more preferably from 0.1 to 10 mm. By adjusting the distance within this range, the refining degree will be improved. Further, it is preferred that such a distance can be adjustable. The distance may be adjusted by moving the roller for precipitation or by moving the scraping member. However, it is preferred to adjust it by moving the scraping member. If the above distance can be adjusted, the size of the solid material to be recovered, can be adjusted, such being desirable. The material for the above scraping member, particularly the material of the forward end, is selected taking the corrosion resistance, etc. into consideration, and a metal material, a ceramics material, glass or a fluorine resin, may, for example, be mentioned as preferred.

The refining device of the present invention preferably has a solid discharge section. The solid discharge section is a section where the precipitated crystals of the solid material are recovered and discharged out of the device. Further, the solid discharge section is preferably provided below the above-mentioned scraping member, so that crystals scraped off from the roller for precipitation at the scraping section will fall and will be led to the solid discharge section. The solid discharge section preferably has a storage section to store the precipitated crystals of the solid material and a discharge means to discharge the stored crystals. The discharge means is preferably a means which is capable of discharging the crystals batch-wisely or continuously. As such a discharge means, a tank having upper and lower two valves and equipped with a line capable of adjusting the pressure, provided at a lower portion of the storage section, may, for example, be mentioned. Here, the valves are valves of a type throughwhich crystals can pass, and ball valves may, for example, be mentioned. Further, it is preferred that the solid discharge section can be cooled.

The refining device of the present invention preferably has a heater to heat the housing. By heating the housing (particularly the top wall and the side walls) by this heater, it is possible to prevent deposition of crystals on the inner wall other than the solid precipitation section in the refining device and to prevent a decrease in the yield. This heater is particularly preferably provided in the vicinity of the bottom surface of the refining device. By providing the heater in the vicinity of the bottom surface, it is possible to heat the entire evaporation section and to vaporize or sublimate the solid material effectively. As a heating source of the heater, an electric heater, steam or electron magnetic induction heating may, for example, be mentioned.

The refining device of the present invention preferably has a pressure reducing means to reduce the pressure in the device. By reducing the pressure in the device, it becomes possible to refine a thermally unstable solid material. As a pressure-reducing means a pressure reducing device connected by a piping to the housing, is preferred. Such a pressure-reducing device preferably has a pressure-adjusting function. As such a pressure reducing device, an oil rotary vacuum pump may, for example, be mentioned. The piping to connect the pressure-reducing device is installed at an optional position in the housing, but it is particularly preferably installed in the solid discharge section. By installing it in the solid discharging section, it is possible to control the vapor of the solid material from flowing into the piping to the pressure reducing device.

The refining device of the present invention preferably has a means to continuously supply the solid material. By having such a continuous supply means, it is possible to supply the solid material continuously into the device, whereby an operation to open the refining device for the supply may be eliminated. Accordingly, inclusion of particles can thereby be prevented. As a continuous supply means, a tank having upper and lower two valves and provided with a line capable of adjusting the pressure, may, for example, be mentioned. It is preferred that this tank can be heated and kept warm. By supplying the solid material as heated, it is possible to improve the efficiency for evaporation or sublimation of the solid material. In such a case, the solid material may be supplied as it is in the form of a solid, or may be supplied in a molten state.

The solid material which can be refined by the present invention is not particularly limited so long as it is a solid material which can be evaporated or sublimated under certain temperature and pressure conditions and which can be precipitated as solid on the roller for precipitation. As such a solid material, an aromatic compound such as phthalic anhydride or naphthalene; a perfluoroaromatic compound such as naphthalene fluoride; a polycyclic quinone pigment; a chelate complex compound; a phthalocyanine dye compound; or a simple substance such as As, I, Al, Sn, Pb or Zn, may be mentioned. As the chelate complex compound, aluminum tris(8-quinolinolate), aluminum acetyl acetonate, or N,N'-diphenyl-N,N'-bis-(3-methylphenyl)-(1,1'-biphenyl)-4,4'-diamine may, for example, be mentioned.

The process for refining the solid material of the present invention is carried out by means of the above-described refining device as follows. Namely, the solid material deposited on the surface of the rotatably installed roller for evaporation, is evaporated or sublimated batch-wisely or continuously. The evaporated or sublimated material is batch-wisely or continuously precipitated on the surface of the rotatably installed roller for precipitation. The crystals precipitated on the surface of the roller for precipitation are batch-wisely or continuously scraped off by the scraping member, and the crystals are batch-wisely or continuously discharged.

In the above refining process, the solid material may be introduced into the refining device in the form of solid, or may be melted and then introduced into the refining device. Introduction of the solid material into the refining device may be carried out batch-wisely or continuously. In such a case, it is preferred to continuously introduce it by means of the above-mentioned continuous supply means. The amount of the solid material to be introduced is preferably to such an extent that the lowermost end of the roller for evaporation is embedded in the solid material introduced into the refining device. Namely, it is preferred that the solid material pooled at the bottom portion of the refining device is controlled to be an amount such that it will be in contact with the roller for evaporation but will not be in contact with the rotational shaft of the roller for evaporation. It is thereby possible to carry out evaporation or sublimation from the surface of the roller for evaporation, and it is possible to obtain such an effect that the roller for evaporation will stir the pooled solid material, such being desirable.

Now, the refining device of the present invention will be described in detail with reference to Fig. 1. Fig. 1 is a vertical cross-sectional view of the essential part of the refining device of the present invention (schematic view of one embodiment).

The refining device comprises a housing 1, an evaporation section 2, a solid precipitation section 3, a scraping section 4, a solid discharge section 5 and a heater 6. The evaporation section 2, the solid precipitation section 3 and the scraping section 4 are disposed in the housing 1. Further, the solid discharge portion 5 is provided in continuation from the housing 1. At a lower portion of the housing 1, a roller 22 for evaporation is disposed, and a roller 32 for precipitation is disposed diagonally above the roller 22 for evaporation. The housing 1 has a bottom wall 10, side walls 11 and 12 and a top wall 13.

The evaporation section 2 is constituted by a space separated from a storage section 51 by a depressed section 7 and is provided with the roller 22 for evaporation. The roller 22 for evaporation is cylindrical and rotates about a rotational axis 21 of the roller for evaporation. The rotational axis 21 of the roller for evaporation is supported by side walls not shown, of the housing 1. The solid precipitation section 3 is constituted by the upper space in the housing 1 and is provided with a roller 32 for precipitation. The roller 32 for precipitation is cylindrical and rotates about a rotational axis 31 of the roller for precipitation. The rotational axis 31 of the roller for precipitation is supported by side walls not shown, of the housing 1.

The scraping section 4 is provided with a scraper 41. The scraper 41 is fixed to the top wall 13 of the housing 1 and has a forward end 42. The forward end 42 is formed to face the roller 32 for precipitation. The forward end 42 and the roller 32 for precipitation are disposed to have a space therebetween. The solid discharge section is provided with a storage section 51 and a discharge section 53. The discharge section 53 is provided below the storage section 51. An upper discharge valve 52 is provided between the storage section 51 and the discharge section 53. Below the discharge section 53, a lower discharge valve 55 is provided. At the discharge section 53, a pressure-adjusting line 54 is provided. The storage section 51 is formed below the scraping section 4 next to a crystal guide wall 61 with a reduced diameter.

Heaters 6 are disposed in the vicinity of the bottom wall 10, the side wall 11 and the top wall 13 of the housing 1. A pressure-reducing device connected to the pressure-adjusting line 54, a continuous supply section, roller driving sections (for evaporation and for precipitation), an internal heating device for the roller 22 for evaporation, and an internal temperature-adjusting device for the roller for precipitation, are not shown.

Now, the refining process of the present invention will be described in detail with reference to Fig. 1. A material 14 which is crude crystals of the solid material to be refined, is introduced into the housing 1 via a continuous supply section not shown. The amount of the material 14 to be introduced, is adjusted to be such a level that the lowermost end of the roller 22 for evaporation is embedded in the material 14. By the heaters provided in the vicinity of the bottom wall 10, the side wall 11 and the top wall 13 of the housing 1, the bottom wall 10, the side wall 11 and the top wall 13 are heated. As the bottom wall 10 is heated, the material 14 is heated and evaporated or sublimated. Further, as the side wall 11 and the top wall 13 are heated, a decrease in the recovery rate due to precipitation of the evaporated or sublimated material 14 on the inner wall surface of the device, can be prevented. Here, it is preferred that the temperature of the side wall 11 and the top wall 13 is controlled to be higher than the evaporation section 2 (specifically the roller 22 for evaporation).

The roller 22 for evaporation is rotated by a driving section not shown. The roller 22 for evaporation is rotated, so that at a lower portion thereof, the material 14 is deposited on the surface of the roller 22 for evaporation. The roller 22 for evaporation is heated by an internal heating device not shown. The heated material 14 on the surface of the roller 22 for evaporation will be evaporated or sublimated. As the roller 22 for evaporation is rotated, the material 14 is stirred, whereby the material 14 can be continuously and constantly (without abrupt boiling or the like) evaporated or sublimated in a large amount.

The roller 32 for precipitation is rotated by a driving section not shown. Further, the roller 32 for precipitation is controlled to be at a temperature slightly lower than the evaporation section 2 (specifically the roller 22 for evaporation). Specifically, the temperature of the roller 32 for precipitation is preferably controlled to be lower by from 5 to 50°C than the roller 22 for evaporation. However, it is preferred that the temperature of the roller 32 for precipitation is controlled to be not more than the melting point of the material 14. The material evaporated or sublimated from the evaporation section 2 including the roller 22 for evaporation, is cooled at the surface of the roller 32 for precipitation and solidified (crystallized) and precipitated. As the roller 32 for precipitation is rotated, crystals will be precipitated uniformly on the surface of the roller 32 for precipitation. The precipitation rate of crystals can be adjusted by the pressure in the device, the temperature of the evaporation section 2, the temperature of the roller 32 for precipitation, the rotational speed of the roller 22 for evaporation and the rotational speed of the roller 32 for precipitation.

The crystals precipitated on the surface of the roller 32 for precipitation, will be scraped off by the forward end 42 of the scraper 41. As the roller 32 for precipitation is rotated, the crystals will be scraped off continuously by the scraper 41. Further, no more than a certain amount of crystals will be deposited on the surface of the roller 32 for precipitation. Further, the surface of the roller 32 for precipitation and the forward end 42 of the scraper 41 are not in contact with each other, whereby inclusion of particles which otherwise takes place by their sliding contact, can be prevented.

Crystals scraped off by the scraper 41, will fall and will be led to the storage section 51 along the crystal guide wall 61. The crystals fallen into the storage section 51, will be discharged out of the device in the following manner. Firstly, usually in operation, i.e. in a step of recovering the crystals in the storage section 51 and the discharge section 53, the upper discharge valve 52 is left open, and the lower discharge valve 55 is closed. By the pressure-reducing device connected to the pressure-adjusting line 54, the pressure in the housing 1 is controlled. In this step, the scraped crystals will be stored in the discharge section 53. In the step of discharging the crystals out of the device, the upper discharge valve 52 is closed, the pressure in the discharge section 53 and outside the device is made equal through the pressure-adjusting line, whereupon the lower discharge valve 55 is opened to discharge crystals out of the device. Here, it is preferred to utilize an inert gas to make the pressure equal. After discharging the crystals out of the device, the lower discharge valve 55 will be closed, and if necessary, the atmosphere in the discharge section 53 will be.replaced by an inert gas, and the pressure in the device and the pressure in the discharge section 53 are made to be equal through the pressure-adjusting line, whereupon the upper discharge valve 52 will be opened, to return to the step of recovering the crystals.

As an inert gas, nitrogen or argon may, for example, be mentioned. Further, it is preferred to provide a filter in the pressure-adjusting line. By providing such a filter, it is possible to prevent inclusion of particles in crystals, and it is possible to prevent the particles from reaching the pressure reducing device through the pressure-adjusting line. Here, it is preferred that the opening of the filter is fine, specifically it is preferably at most 0.1 µm, more preferably at most 0.02 µm, particularly preferably at most 0.003 µm.

The above-described operation for discharging the crystals out of the device will not hinder the evaporation or sublimation at the evaporation section 2 or the precipitation of crystals at the roller 32 for precipitation. Accordingly, a continuous refining operation is possible, whereby the refining efficiency is high. Further, the crystals can be discharged out of the device with only the necessary minimum opening operation, whereby inclusion of particles can be suppressed.

### EXAMPLES

Now, the refining process employing the refining device of the present invention will be described with reference to Examples.

The refining device employed, has the structure shown in Fig. 1. The size of the housing 1 is such that the long axial direction of the roller 22 for evaporation is 38 cm, the width (the left-right direction on the paper surface) is 43 cm, and the height is 30 cm. However, this size does not include a section lower than the upper discharge valve 52. The housing 1 is made of stainless steel (SUS316). The diameter of the roller 22 for evaporation is 15.8 cm, its longitudinal direction is 15 cm, and the material is made of stainless steel (SUS316). Further, the diameter of the roller 32 for precipitation is 13.4 cm, its longitudinal direction is 15 cm, and the material is made of stainless steel (SUS316). The distance between the rotational axis 21 of the roller for evaporation and the rotational axis 31 of the roller for precipitation is 15.6 cm (the shortest distance between the outer peripheries of the two rollers is 1.0 cm).

Refining of each of the following samples was carried out by using the above-described refining device unless otherwise specifically mentioned. The refined purity of the sample was measured by a gas chromatography method. The refining conditions and the results of refining (purities) are shown in Tables 1 and 2. In the following description, "the pressure in the system" means the pressure in the housing during the refining operation. Further, "the temperature for heating the material" means the temperature for heating the solid material at the initial stage of the refining operation, and the temperature of the bottom wall 10 was measured. Further, "the heating maintenance temperature" means the temperature at which the solid material is maintained at a prescribed temperature in the intermediate stage (the stage after the initial stage) of the refining operation, and the temperature of the bottom wall 10 was measured. Further, "the roller heating temperature" means the temperature of the roller 32 for precipitation at the initial stage of the refining operation, and "the roller maintenance temperature" means the temperature of the roller 32 for precipitation at the intermediate stage of the refining operation. Further, the unit (rpm) of the rotation speed of the roller means the number of rotations per minute.

### EXAMPLE 1

120 g of non-refined perfluoro(1,3,5-triphenylbenzene) (hereinafter referred to as "TPB") having a purity of 96.1%, was introduced into the lower portion of the roller 22 for evaporation. The pressure in the system was adjusted to and maintained at 400 Pa by means of an oil-sealed rotary vacuum pump and a pressure-adjusting device. Heating was carried out from the exterior by a heater, whereby the temperature for heating the material was set to be 165°C. Then, the heating maintenance temperature was adjusted to be 160°C. The roller 22 for evaporation was rotated at a rotational speed of 20 rpm, and the rotational direction was clockwise in Fig. 1. The temperature of the roller 22 for evaporation was controlled within a range of from the temperature for heating the material or the same temperature as the heating maintenance temperature, to a temperature higher by 5°C. The roller 32 for precipitation was rotated at a rotational speed of 3 rpm, and the rotational direction was anticlockwise in Fig. 1. The roller heating temperature was adjusted to be 115°C, and the roller maintenance temperature was also adjusted to be 115°C. After a few minutes from the initiation of the heating, crystals started to precipitate on the roller 32 for precipitation. After 4.5 hours from the initiation of the heating, 56 g of the desired white columnar crystals were recovered. The purity of the recovered refined TPB was 99.96%.

### EXAMPLE 2

120 g of the same TPB as in Example 1 was introduced into the refining device. Refining was carried out under the same conditions as in Example 1 except that the pressure in the system was changed to 133 Pa. After 3 hours from the initiation of the heating, 81 g of desired white columnar crystals were recovered. The purity of the recovered refined TPB was 99.91%.

### EXAMPLE 3

150 g of the same TPB as in Example 1 was introduced into the refining device. After a few minutes from the initiation of the heating, crystals started to precipitate on the surface of the roller 32 for precipitation. Here, preliminarily molten non-refined TPB having the same purity was continuously supplied to the lower portion of the roller for evaporation at a rate of 14.5 g/hr by means of a pump. Refining was carried out under the same conditions as in Example 1 except that the material was continuously supplied. After 8.5 hours from the initiation of the heating, 123 g of desired white columnar crystals were continuously recovered. The purity of the recovered refined TPB was 99.98%.

### EXAMPLE 4

A roller for evaporation, having a helical groove formed in a depth of 5 mm on the surface to have a shape to facilitate deposition of the solid, was mounted on the refining device. 300 g of the same TPB as in Example 1 was introduced into the refining device. The temperature for heating the material and the material maintenance temperature were adjusted to be 145°C, and the pressure in the system was adjusted to be 13.0 Pa. Otherwise, purification was carried out under the same conditions as in Example 1. After 8.5 hours from the initiation of the heating, 123 g of the desired white columnar crystals, was recovered. The purity of the recovered refined TPB was 99.98%.

Further, the vapor pressure of TPB was 400 Pa at 145°C, and the melting point is 152°C.

### EXAMPLE 5

150 g of non-refined fluorinated naphthalene (hereinafter referred to as "FNP") was introduced at the lower portion of the roller 22 for evaporation. The pressure in the system was adjusted to be 1333 Pa. The temperature for heating the material and the heating maintenance temperature were adjusted to be 105°C, and the roller heating temperature and the roller maintenance temperature were adjusted to be 75°C. Further, the roller 22 for evaporation was rotated at a rotational speed of 25 rpm, and the roller 32 for precipitation was rotated at a rotational speed of 5 rpm. The rotational directions were the same directions as in Example 1. The material was supplied continuously in the same manner as in Example 3. After 7.5 hours from the initiation of the heating, 108 g of desired needle crystals were continuously recovered. The purity of the recovered refined FNP was 99.91%.

### EXAMPLE 6

120 g of non-refined perfluoro(2,4,6-triphenyltriazine) (hereinafter referred to as "TPT") having a purity of 91.3%, was introduced at the lower portion of the roller 22 for evaporation. The pressure in the system was adjusted to be 667 Pa. The temperature for heating the material and the heating maintenance temperature were adjusted to be 135°C, and the roller heating temperature and the roller maintenance. temperature were adjusted to be 105°C. Further, the roller 22 for evaporation was rotated at a rotational speed of 20 rpm, and the roller 32 for precipitation was rotated at a rotational speed of 5 rpm. The rotational directions were the same directions as in Example 1. After 6 hours from the initiation of the heating, 89 g of desired crystals were recovered. The purity of the recovered refined TPT was 99.89%.

### EXAMPLE 7

200 g of non-refined fluorinated biphenyl (hereinafter referred to as "FBP") was introduced at the lower portion of the roller 22 for evaporation. The pressure in the system was adjusted to be 1333 Pa. The temperature for heating the material and the heating maintenance temperature were adjusted to be 85°C, and the roller heating temperature and the roller maintenance temperature were adjusted to be 55°C. Further, the roller 22 for evaporation was rotated at a rotational speed of 20 rpm, and the roller 32 for precipitation was rotated at a rotational speed of 5 rpm. The rotational directions were the same directions as in Example 1. The material was continuously supplied in the same manner as in Example 3. After 8.0 hours from the initiation of the heating, 148 g of desired needle crystals were continuously recovered. The purity of the recovered refined FBP was 98.7%.

### EXAMPLE 8

In the refining device, the distance between the surface of the roller 22 for evaporation and the surface of the roller for precipitation was adjusted to be 20 mm. 150 g of non-refined anthracene was introduced at the lower portion of the roller 22 for evaporation. The pressure in the system was adjusted to be from 267 to 400 Pa. The temperature for heating the material and the heating maintenance temperature were adjusted to be 210°C, and the roller heating temperature and the roller maintenance temperature were adjusted to be 180°C. Further, the roller 22 for evaporation was rotated at a rotational speed of 15 rpm, and the roller 32 for precipitation was rotated at a rotational speed of 5 rpm. The rotational directions were the same directions as in Example 1. After 8.5 hours from the initiation of the heating, 102 g of desired needle crystals were recovered. The purity of the recovered refined anthracene was 99.95%.

### EXAMPLE 9

150 g of non-refined naphthalene was introduced at the lower portion of the roller 22 for evaporation. The pressure in the system was adjusted to be 2667 Pa. The temperature for heating the material and the heating maintenance temperature were adjusted to be 85°C, and the roller heating temperature and the roller maintenance temperature were adjusted to be 70°C. Further, the roller 22 for evaporation was rotated at a rotational speed of 15 rpm, and the roller 32 for precipitation was rotated at a rotational speed of 5 rpm. The rotational directions were the same directions as in Example 1. After 8.5 hours from the initiation of the heating, 102 g of desired white needle crystals were recovered. The purity of the recovered refined naphthalene was 99.0%.

### EXAMPLE 10

150 g of the same naphthalene as in Example 9 was introduced into the refining device. Refining was carried out under the same conditions as in Example 9 except that in the refining device, the distance between the surface of the roller 22 for evaporation and the surface of the roller for precipitation was adjusted to be 15 mm. After 8.5 hours from the initiation of the heating, 106 g of desired white needle crystals were recovered. The purity of the recovered refined naphthalene was 99.2%.

### EXAMPLE 11

A roller for evaporation having a helical groove formed in a depth of 5 mm on the surface to have a shape to facilitate the deposition of the solid, was mounted on the refining device. 200 g of non-refined aluminum tris(8-quinolinolate) (hereinafter referred to as "TQA") was introduced at the lower portion of the roller 22 for evaporation. The pressure in the system was adjusted to be 400 Pa. The temperature for heating the material and the heating maintenance temperature were adjusted to be 310°C, and the roller heating temperature and the roller maintenance temperature were adjusted to be 280°C. Further, the roller 22 for evaporation was rotated at a rotational speed of 15 rpm, and the roller 32 for precipitation was rotated at a rotational speed of 5 rpm. The rotational directions were the same direction as in Example 1. After 8 hours from the initiation of the heating, 113 g of desired refined crystals were recovered. The purity of the recovered refined TQA was 99.5%.

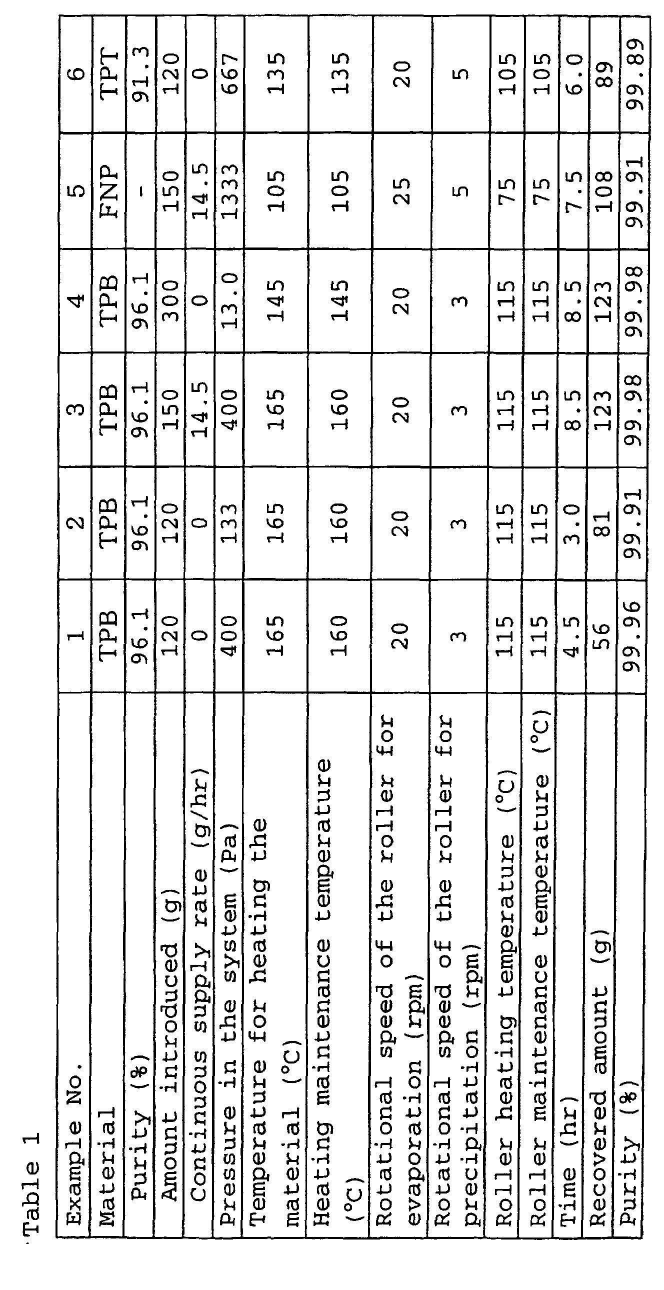

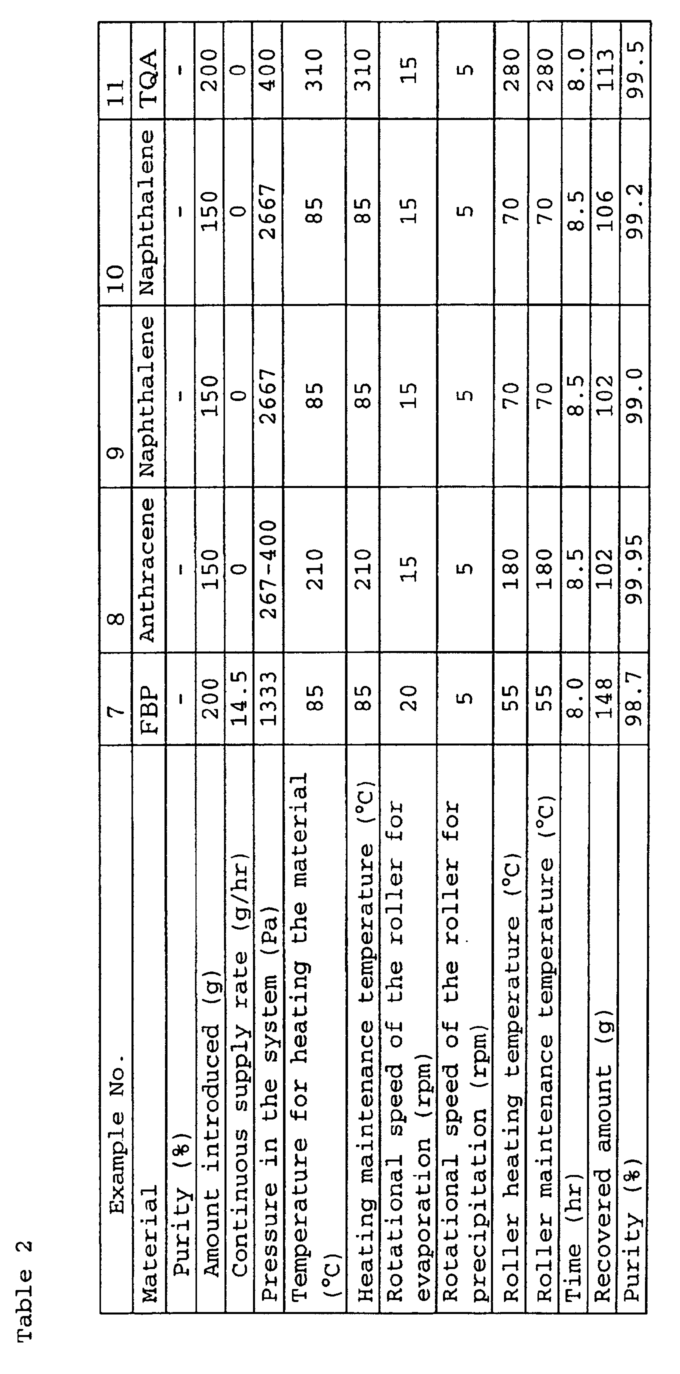

### INDUSTRIAL APPLICABILITY

According to the device for refining a solid material of the present invention and the refining process employing such a refining device, from the supply of the material to the operation for recovering crystals, can be carried out batch-wisely or continuously in a series of operations, whereby a solid material of high purity can be obtained highly efficiently.

Namely, the refining process of the present invention makes refining in high purity possible as compared with refining by distillation or recrystallization which is carried out as a common refining process. Further, the refining device of the present invention is simple in structure and is capable of preventing inclusion of impurities attributable to the interior of the device. Further, an opened operation of the entire device is not required, whereby inclusion of particles can be prevented.

The refining process of the present invention is useful as a process for refining a material, where a high level of refining and exclusion of impurities are required, such as an electronic material or an optical material.

## Claims

1. A device for refining an evaporable or sublimable solid material, which comprises a housing, and at least one rotatable roller for evaporation and at least one rotatable roller for precipitation, installed in the housing.

2. The device for refining the solid material according to Claim 1, wherein the distance between the roller for evaporation and the roller for precipitation is adjustable.

3. The device for refining the solid material according to Claim 1 or 2, wherein the surface of the roller for evaporation and/or the roller for precipitation has an irregularity.

4. The device for refining the solid material according to any one of Claims 1 to 3, wherein the roller for evaporation and the roller for precipitation can be heated and/or cooled.

5. The device for refining the solid material according to any one of Claims 1 to 4, which is further provided with a scraping means in the vicinity of the roller for precipitation, wherein there is a space left between the surface of the roller for precipitation and the forward end of the scraping means.

6. The device for refining the solid material according to any one of Claims 1 to 5, wherein the housing is heated to prevent deposition of crystals onto the inner wall of the housing.

7. A process for refining a solid material, which comprises:
batch-wisely or continuously evaporating or sublimating a solid material deposited on the surface of a rotatably installed roller for evaporation,
batch-wisely or continuously precipitating the evaporated or sublimated material on a rotatably installed roller for precipitation,
batch-wisely or continuously scraping off crystals precipitated on the surface of the roller for precipitation, at a scraping section, and
batch-wisely or continuously discharging the crystals.
